# EUROPEAN PATENT SPECIFICATION

(11) **EP 1 466 988 B1**
(45) Date of publication and mention of the grant of the patent: **27.02.2013**
(21) Application number: 04015121.9
(22) Date of filing: 10.06.1999
(51) Int. Cl.: C12Q 1/68, C12N 15/11, G01N 33/574, A61P 35/00

(54) **Genes and gene expression products that are differentially regulated in prostate cancer**
Gene und gen-expressions Produkte die in Prostatakrebs differentiel-reguliert sind
Gènes et produits d'expression génique régulés de façon différentielle lors du cancer de la prostate

(30) Priority: 11.06.1998 US 88877 P; 09.06.1999 US 328475
(43) Date of publication of application: 13.10.2004
(62) Divisional of application: 99927460.8
(73) Proprietor: Novartis Vaccines and Diagnostics, Inc., Emeryville, CA 94608 (US)
(72) Inventor: Astel, Jon H., Emeryville California 94608-2916 (US); Carroll, Eddie, III., Emeryville California 94608-2916 (US); Endege, Wilson O., Emeryville California 94608-2916 (US); Ford, Donna M., Emeryville California 94608-2916 (US); Monahan, John E., Emeryville California 94608-2916 (US); Schlegel, Robert, Emeryville California 94608-2916 (US); Steinmann, Kathleen E., Emeryville California 94608-2916 (US); Zhang, Jimmy, Emeryville California 94608-2916 (US)
(74) Representative: Woods, Geoffrey Corlett

(56) References cited:
- WO-A-00/12529
- WO-A-00/29017
- WO-A-92/03127
- WO-A-98/04689
- DATABASE UniProt [Online] 1 July 1997 (1997-07-01), "Nonstructural protein 1 (Fragment)." XP002310972 retrieved from EBI accession no. UNIPROT:O10254 Database accession no. O10254
- DATABASE UniProt [Online] 1 November 1996 (1996-11-01), "Protein tyrosine phosphatase epsilon cytoplasmic isoform (Fragment)." XP002310973 retrieved from EBI accession no. UNIPROT:Q13345 Database accession no. Q13345
- HELLER R A ET AL: "Discovery and analysis of inflammatory disease-related genes using cDNA microarrays" PROCEEDINGS OF THE NATIONAL ACADEMY OF SCIENCES OF USA, no. 94, March 1997 (1997-03), pages 2150-2155, XP002076789 ISSN: 0027-8424
- DATABASE EMBL [Online] 12 March 1999 (1999-03-12), "Homo sapiens mRNA; EST DKFZp434B083_s1 (from clone DKFZp434B083)" XP002310974 retrieved from EBI accession no. EM_PRO:AL044841 Database accession no. AL044841

## Description

### FIELD OF THE INVENTION

This invention relates to the area of diagnosis and prognosis of cancer, tumor progression, hyperproliferative cell growth, and accompanying physical and biological manifestations. More specifically, the invention includes polynucleotides that are differentially regulated in prostatic disorders, such as metastatic prostate cancer, localized prostate cancer, and benign prostate hyperplasia (BPH).

### BACKGROUND OF THE INVENTION

Genes that are up-or down-regulated in cancer or tumor progression are useful for therapeutic and diagnostic purposes. For example, detection of genes or gene expression products up-regulated in hyperproliferative cells can be a predictive or diagnostic marker of the onset or the progression of cancer. Early diagnosis can be useful if the cancer, tumors, or hyperproliferating cells can be inhibited, removed, or terminated to prevent metastasis or recurrence of cancerous growth. Such early warning is of particular use to prostate cancer patients, where removal of the growth, tumor, or cells is beneficial if the disease is confined to the prostate. There is a need in the art for genes related to cancer and tumor progression.

### SUMMARY OF THE INVENTION

The present invention provides methods and reagents for diagnosing prostate cancer, prostate tumor progression or hyperproliferative prostate cell growth. Reagents for such diagnostic kits include polynucleotides comprising at least 20 contiguous nucleotides of SEQ ID NOS: 218, 219 or 335 or a complement thereof.

The invention provides:
- a method of diagnosing prostate cancer, prostate tumour progression and hyperproliferative prostate cell growth comprising:
   (a) providing a polynucleotide probe comprising at least 20 contiguous nucleotides of SEQ ID NO: 218, 219 or 335 or a complement thereof;
   (b) contacting a biological sample for diagnosis with said probe under hybridizing conditions that permit formation of a duplex; and
   (c) determining the presence of said duplex, and detecting the differences that exist between the levels of duplexes in said biological sample as compared to a normal biological sample.
- A diagnostic kit comprising:
   (a) a diagnostic regeant comprising a polynucleotide probe comprising at least 20 contiguous nucleotides of SEQ ID NO: 218, 219 or 335 or a complement thereof when said sequence is present in a test biological sample;
   (b) a normal biological sample; and
   (c) instructions for detecting the differences that exist between the levels of duplexes in said test biological sample as compared to said normal biological sample.
- An isolated polynucleotide selected from the group consisting of: a polynucleotide comprising the nucleotide sequence of SEQ ID NO: 218 or 219.
- A vector comprising the above polynucleotide.
- An isolated host cell comprising the above vector.

The methods of diagnosis described herein include both nucleic acid assays and immunoassays.

Preferably, nucleic acid obtained from biological material is genomic DNA or mRNA. The nucleic acid can also be cDNA complementary to the mRNA.

Also described is the use of the isolated polynucleotides or parts thereof as diagnostic probes or as primers.

In certain preferred embodiments, the polynucleotide is operably linked to an expression control sequence. The invention further provides a host cell, including bacterial, yeast, insect and mammalian cells, transformed with the polynucleotide sequence. The invention also provides the full-length cDNA and the full length human gene corresponding to the polynucleotide.

Also described is nucleic acid obtained by transforming a host cell with nucleic acid comprising at least 20 contiguous nucleotides of SEQ ID NO: 218, 219 or 335, culturing the host cell, and recovering the replicated nucleic acid, the expressed RNA, and/or the expressed polypeptide.

### Detailed Description of the Invention

Genes that are up- or down-regulated in cancer or tumor progression are useful for therapeutic and diagnostic purposes. For example, a diagnostic assay to determine the stage of the disease also is useful in tailoring treatment of aggressive versus more mild cancer or tumor progression. The polynucleotide sequences and encoded polypeptides described herein are useful for these diagnostic or prognostic purposes.

Further, modulation of genes or gene expression products that are mis-regulated can be used to treat or ameliorate cancer, tumor progression, hyperproliferative cell growth, and the accompanying physical and biological manifestations.

Identified herein are polynucleotide sequences that are upregulated in a cancer cell line, more specifically in a prostate cancer cell line. The present invention relates to methods and reagents for diagnosis.

### I. Use of Polynucleotides Having a Sequence of One or More of SEQ ID NO:1-339 to Obtain Full-Length cDNA and Full-Length Human Gene and Promoter Region

Full-length cDNA molecules comprising the disclosed sequences are obtained as follows. The polynucleotide or a portion thereof comprising at least 12, 15, 18, or 20 nucleotides is used as a hybridization probe to detect hybridizing members of a cDNA library using probe design methods, cloning methods, and clone selection techniques as described in U.S. Patent No. 5,654,173, "Secreted Proteins and Polynucleotides Encoding Them," incorporated herein by reference. Libraries of cDNA are made from selected tissues, such as normal or tumor tissue, or from tissues of a mammal treated with, for example, a pharmaceutical agent. Preferably, the tissue is the same as that used to generate the polynucleotides, as both the polynucleotides and the cDNA represent expressed genes. Most preferably, the cDNA library is made from the biological material described herein in the Examples. Alternatively, many cDNA libraries are available commercially. (Sambrook et al., Molecular Cloning: A Laboratory Manual, 2nd Ed (Cold Spring Harbor Press, Cold Spring Harbor, NY 1989).

Members of the library that are larger than the polynucleotide, and preferably that contain the whole sequence of the native message, are obtained. In order to confirm that the entire cDNA has been obtained, RNA protection experiments are performed as follows. Hybridization of a full-length cDNA to an mRNA will protect the RNA from RNase degradation. If the cDNA is not full length, then the portions of the mRNA that are not hybridized will be subject to RNase degradation. This is assayed, as is known in the art, by changes in electrophoretic mobility on polyacrylamide gels, or by detection of released monoribonucleotides. Sambrook et al., Molecular Cloning: A Laboratory Manual, 2nd Ed (Cold Spring Harbor Press, Cold Spring Harbor, NY 1989). In order to obtain additional sequences 5' to the end of a partial cDNA, 5' RACE (PCR Protocols: A Guide to Methods and Applications (Academic Press, Inc. 1990)) is performed.

Genomic DNA is isolated using polynucleotides in a manner similar to the isolation of full-length cDNAs. Briefly, the polynucldotides, or portions thereof, are used as probes to libraries of genomic DNA. Preferably, the library is obtained from the cell type that was used to generate the polynucleotides, but this is not essential. Most preferably, the genomic DNA is obtained from the biological material described herein in the Examples. Such libraries may be in vectors suitable for carrying large segments of a genome, such as P1 or YAC, as described in detail in Sambrook *et al.,* 9.4-9.30. In addition, genomic sequences can be isolated from human BAC libraries, which are commercially available from Research Genetics, Inc., Huntville, Alabama, USA, for example. In order to obtain additional 5' or 3' sequences, chromosome walking is performed, as described in Sambrook *et al.,* such that adjacent and overlapping fragments of genomic DNA are isolated. These are mapped and pieced together, as is known in the art, using restriction digestion enzymes and DNA ligase.

Using the polynucleotides sequences of the invention, corresponding full length genes can be isolated using both classical and PCR methods to construct and probe cDNA libraries. Using either method, Northern blots, preferably, are performed on a number of cell types to determine which cell lines express the gene of interest at the highest rate.

Classical methods of constructing cDNA libraries are taught in Sambrook et al., supra. With these methods, cDNA can be produced from mRNA and inserted into viral or expression vectors. Typically, libraries of mRNA comprising poly(A) tails can be produced with poly(T) primers. Similarly, cDNA libraries can be produced using the instant sequences as primers.

PCR methods are used to amplify the members of a cDNA library that comprise the desired insert. In this case, the desired insert will contain sequence from the full length cDNA that corresponds to the instant ESTs. Such PCR methods include gene trapping and RACE methods. Gruber *et al.,* PCT WO 95/04745 and Gruber *et al.,* U.S. Pat. No. 5,500,356. Kits are commercially available to perform gene trapping experiments from, for example. Life Technologies, Gaithersburg, Maryland, USA. PCT Pub. No. WO 97/19110. (Apte and Siebert, Biotechniques 15:890-893, 1993; Edwards et al., Nuc. Acids Res. 19:5227-5232,1991).

The promoter region of a gene generally is located 5' to the initiation site for RNA polymerase II, and can be obtained by performing 5' RACE using a primer from the coding region of the gene. Alternatively, the cDNA can be used as a probe for the genomic sequence, and the region 5' to the coding region is identified by "walking up." If the gene is highly expressed or differentially expressed, the promoter from the gene may be of use in a regulatory construct for a heterologous gene.

Once the full-length cDNA or gene is obtained, DNA encoding variants can be prepared by site-directed mutagenesis, described in detail in Sambrook *et al.,* 15.3-15.63. The choice of codon or nucleotide to be replaced can be based on disclosure herein on optional changes in amino acids to achieve altered protein structure and/or function.

As an alternative method to obtaining DNA or RNA from a biological material, nucleic acid comprising nucleotides having the sequence of one or more polynucleotides of the invention can be synthesized. Thus, the invention encompasses nucleic acid molecules ranging in length from 20 nucleotides (corresponding to at least 20 contiguous nucleotides of SEQ ID NO: 218, 219 or 335) up to a maximum length suitable for one or more biological manipulations, including replication and expression, of the nucleic acid molecule. The invention includes but is not limited to (a) nucleic acid having the size of a full gene, and comprising at least 20 contiguous nucleotides of SEQ ID NO:218, 219 or 335; (b) the nucleic acid of (a) also comprising at least one additional gene, operably linked to permit expression of a fusion protein; (c) an expression vector comprising (a) or (b); (d) a plasmid comprising (a) or (b) ; and (e) a recombinant viral particle comprising (a) or (b).

The sequence of a nucleic acid comprising at least 20 contiguous nucleotides of at least any one of SEQ ID NO: 218, 219 or 335, preferably the entire sequence of at least any one of SEQ ID NO: 218, 219 or 335, is not limited and can be any sequence of A, T, G, and/or C (for DNA) and A, U, G, and/or C (for RNA) or modified bases thereof, including inosine and pseudouridine. The choice of sequence will depend on the desired function and can be dictated by coding regions desired, the intron-like regions desired, and the regulatory regions desired.

Where the entire sequence of any one of SEQ ID NO: 218, 219 or 335 is within the nucleic acid, the nucleic acid obtained is referred to herein as a polynucleotide comprising the sequence of any one of SEQ ID NO: 218, 219 or 335.

### II. Expression of Polypeptide Encoded by Full-Length cDNA or Full-Length Gene

The polynucleotide, the corresponding cDNA, or the full-length gene is used to express the partial or complete gene product Appropriate polynucleotide constructs are purified using standard recombinant DNA techniques as described in, for example, Sambrook *et al.,* (1989) *Molecular Cloning: A Laboratory Manual,* 2nd ed. (Cold Spring Harbor Press, Cold Spring Harbor, New York). The polypeptides encoded by the polynucleotides are expressed in any expression system, including, for example, bacterial, yeast, insect, amphibian and mammalian systems. Suitable vectors and host cells are described in U.S. Patent No. 5,654,173.

Bacteria. Expression systems in bacteria include those described in Chang et al., Nature (1978) 275:615, Goeddel et al., Nature (1979) 281:544, Goeddel et al., Nucleic Acids Res. (1980) 8:4057; EP 0 036,776, U.S. Patent No. 4,551,433, DeBoer et al., Proc. Natl. Acad Sci. (USA) (1983) 80:21-25, and Siebenlist et al., Cell (1980) 20:269.

Yeast. Expression systems in yeast include those described in Hinnen et al., Proc. Natl. Acad Sci. (USA) (1978) 75:1929; Ito et al., J. Bacteriol. (1983) 153:163; Kurtz et al., Mol. Cell. Biol. (1986) 6:142; Kunze et al., J. Basic Microbiol. (1985) 25:141; Gleeson et al., J. Gen. Microbiol. (1986) 132:3459, Roggenkamp et al., Mol. Gen. Genet. (1986) 202:302) Das et al., J. Bacteriol. (1984) 158:1165*;* De Louvencourt et al., J. Bacteriol. (1983) 154:737, Van den Berg et al., BiolTechnology (1990) 8:135; Kunze et al., J. Basic Microbiol. (1985) 25:141; Cregg et al., Mol. Cell. Biol. (1985) 5:3376, U.S. Patent Nos. 4,837,148 and 4,929,555; Beach and Nurse, Nature (1981) 300:706; Davidow et al., Curr. Genet. (1985) 10:380, Gaillardin et al., Curr. Genet. (1985) 10:49, Ballance et al., Biochem. Biophys. Res. Commun. (1983) 112:284-289; Tilburn et al., Gene (1983) 26:205-221, Yelton et al., Proc. Natl. Acad Sci. (USA) (1984) 81:1470-1474, Kelly and Hynes, EMBO J. (1985) 4:475479; EP 0 244,234, and WO 91/00357.

Insect Cells. Expression of heterologous genes in insects is accomplished as described in U.S. Patent No. 4,745,051, Friesen et al. (1986) "The Regulation of Baculovirus Gene Expression" in: The Molecular Biology Of Baculoviruses (W. Doerfler, ed.), EP 0 127,839, EP 0 155,476, and Vlak et al., J. Gen. Virol. (1988) 69:765-776, Miller et al., Ann. Rev. Microbiol. (1988) 42:177, Carbonell et al., Gene (1988) 73:409, Maeda et al., Nature (1985) 315:592-594, Lebacq-Verheyden et al., Mol. Cell. Biol. (1988) 8:3129; Smith et al., Proc. Natl. Acad. Sci. (USA) (1985) 82:8404, Miyajima et al., Gene (1987) 58:273; and Martin et al., DNA (1988) 7:99. Numerous baculoviral strains and variants and corresponding permissive insect host cells from hosts are described in Luckow et al., Bio/Technology (1988) 6:47-55, Miller et al., Generic Engineering (Setlow, J.K. et al. eds.), Vol. 8 (Plenum Publishing, 1986), pp. 277-279, and Maeda et al., Nature, (1985) 315:592-594.

Mammalian Cells. Mammalian expression is accomplished as described in Dijkema et al., EMBO J. (1985) 4:761, Gorman et al., Proc. Natl. Acad Sci. (USA) (1982) 79:6777, Boshart et al., Cell (1985) 41:521 and U.S. Patent No. 4,399,216. Other features of mammalian expression are facilitated as described in Ham and Wallace, Meth. Enz. (1979) 58:44, Barnes and Sato, Anal. Biochem. (1980) 102:255, U.S. Patent Nos. 4,767,704, 4,657,866, 4,927,762, 4,560,655, WO 90/103430, WO 87/00195, and U.S. RE 30,985.

Polynucleotide molecules comprising the polynucleotide sequence are propagated by placing the molecule in a vector. Viral and non-viral vectors are used, including plasmids. The choice of plasmid will depend on the type of cell in which propagation is desired and the purpose of propagation. Certain vectors are useful for amplifying and making large amounts of the desired DNA sequence. Other vectors are suitable for expression in cells in culture. Still other vectors are suitable for transfer and expression in cells in a whole animal or person. The choice of appropriate vector is well within the skill of the art. Many such vectors are available commercially. The polynucleotide is inserted into a vector typically by means of DNA ligase attachment to a cleaved restriction enzyme site in the vector. Alternatively, the desired nucleotide sequence may be inserted by homologous recombination in vivo. Typically this is accomplished by attaching regions of homology to the vector on the flanks of the desired nucleotide sequence. Regions of homology are added by ligation of oligonucleotides, or by polymerase chain reaction using primers comprising both the region of homology and a portion of the desired nucleotide sequence, for example.

Polynucleotides are linked to regulatory sequences as appropriate to obtain the desired expression properties. These may include promoters (attached either at the 5' end of the sense strand or at the 3' end of the antisense strand), enhancers, terminators, operators, repressors, and inducers. The promoters may be regulated or constitutive. In some situations it may be desirable to use conditionally active promoters, such as tissue-specific or developmental stage-specific promoters. These are linked to the desired nucleotide sequence using the techniques described above for linkage to vectors. Any techniques known in the art may be used.

When any of the above host cells, or other appropriate host cells or organisms, are used to replicate and/or express the polynucleotides or nucleic acids of the invention, the resulting replicated nucleic acid, RNA, expressed protein or polypeptide, is a product of the host cell or organism. The product is recovered by any appropriate means known in the art.

Once the gene corresponding to the polypeptide is identified, its expression can be regulated in the cell to which the gene is native. For example, an endogenous gene of a cell can be regulated by an exogenous regulatory sequence as disclosed in U.S. Patent No. 5,641,670, "Protein Production and Protein Delivery."

### Identification of Secreted and Membrane-Bound Polypeptides

Both secreted and membrane-bound polypeptides are of interest. For example, levels of secreted polypeptides can be assayed conveniently in body fluids, such as blood, urine, prostatic fluid and semen. Membrane-bound polypeptides are useful for constructing vaccine antigens or inducing an immune response. Such antigens would comprise all or part of the extracellular region of the membrane-bound polypeptides.

Because both secreted and membrane-bound polypeptides comprise a fragment of contiguous hydrophobic amino acids, hydrophobicity predicting algorithms can be used to identify such polypeptides.

A signal sequence is usually encoded by both secreted and membrane-bound polypeptide genes to direct a polypeptide to the surface of the cell. The signal sequence usually comprises a stretch of hydrophobic residues. Such signal sequences can fold into helical structures.

Membrane-bound polypeptides typically comprise at least one transmembrane region that possesses a stretch of hydrophobic amino acids that can transverse the membrane. Some transmembrane regions also exhibit a helical structure.

Hydrophobic fragments within a polypeptide can be identified by. using computer algorithms. Such algorithms include Hopp & Woods, Proc. Natl. Acad. Sci. USA 78: 3824-3828 (1981); Kyte & Doolittle, J. Mol. Biol. 157: 105-132 (1982); and RAOAR algorithm, Degli Esposti et al., Eur. J. Biochem. 190: 207-219 (1990).

Another method of identifying secreted and membrane-bound polypeptides is to translate the polynucleotides described herein, in all six frames and determine if at least 8 contiguous hydrophobic amino acids are present. Those translated polypeptides with at least 8; more typically, 10; even more typically, 12 contiguous hydrophobic amino acids are considered to be either a putative secreted or membrane bound polypeptide. Hydrophobic amino acids include alanine, glycine, histidine, isoleucine, leucine, lysine, methionine, phenylalanine, proline, threonine, tryptophan, tyrosine, and valine.

Putative secreted and/or membrane-bound polypeptides are encoded by the sequences of the following clones: SL-5, SL-6,-SL-9, SL-11, SL-13, SL-90, SL-100, SL-107, SL-124, SL-135, SL-139, SL-143, SL-152, SL-153, SL-173, and SL-177.

### Construction of Polypeptides and Variants Thereof

The polypeptides described herein include those encoded by the disclosed polynucleotides. These polypeptides can also be encoded by nucleic acids that, by virtue of the degeneracy of the genetic code, are not identical in sequence to the disclosed polynucleotides. Thus, described herein are nucleic acids comprising polynucleotides encoding a protein or polypeptide expressed by a polynucleotide of the invention. Also described are variants; variants of polypeptides include mutants, fragments, and fusions. Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge, hydrophobicity/hydrophilicity, and/or steric bulk of the amino acid substituted. For example, substitutions between the following groups are conservative: Gly/Ala, Val/Ile/Leu, Asp/Glu, Lys/Arg, Asn/Gln, Ser/Cys,Thr, and Phe/Trp/Tyr.

Cysteine-depleted muteins are variants within the scope of the invention. These variants can be constructed according to methods disclosed in U.S. Patent No. 4,959,314, "Cysteine-Depleted Muteins of Biologically Active Proteins." The patent discloses how to substitute other amino acids for cysteines, and how to determine biological activity and effect of the substitution. Such methods are suitable for proteins as described herein that have cysteine residues suitable for such substitutions, for example to eliminate disulfide bond formation.

The protein variants described herein are encoded by polynucleotides described herein. The genetic code can be used to select the appropriate codons to construct the corresponding variants.

The invention encompasses polynucleotide sequences having at least 65% sequence identity to and at least 20 contignous nucleotides of SEQ ID NOs: 218, 219 or 335 as determined by the Smith-Waterman homology search algorithm as implemented in MSPRCH program (Oxford Molecular) using an affine gap search with the following search parameters: gap open penalty of 12, and gap extension penalty of 1.

### Use of the Polynucleotides as Probes, in Mapping, and in Tissue Profiling

### Probes

Polynucleotide probes comprising at least 12 contiguous nucleotides selected from the nucleotide sequence of a polynucleotide of the invention are used for a variety of purposes, including identification of human chromosomes and determining transcription levels.

The nucleotide probes are labeled, for example, with a radioactive, fluorescent, biotinylated, or chemiluminescent label, and detected by well known methods appropriate for the particular label selected. Protocols for hybridizing nucleotide probes to preparations of metaphase chromosomes are also well known in the art. A nucleotide probe will hybridize specifically to nucleotide sequences in the chromosome preparations which are complementary to the nucleotide sequence of the probe. A probe that hybridizes specifically to a polynucleotide should provide a detection signal at least 5-, 10-, or 20-fold higher than the background hybridization provided with other unrelated sequences.

In a non-limiting example, commercial programs are available for identifying regions of chromosomes commonly associated with disease, such as cancer. Polynucleotides of the invention can be used to probe these regions. For example, if through profile searching a polynucleotide is identified as corresponding to a gene encoding a kinase, its ability to bind to a cancer-related chromosomal region will suggest its role as a kinase in one or more stages of tumor cell development/growth. Although some experimentation would be required to elucidate the role, the polynucleotide constitutes a new material for isolating a specific protein that has potential for developing a cancer diagnostic or therapeutic.

Nucleotide probes are used to detect expression of a gene corresponding to the polynucleotide. For example, in Northern blots, mRNA is separated electrophoretically and contacted with a probe. A probe is detected as hybridizing to an mRNA species of a particular size. The amount of hybridization is quantitated to determine relative amounts of expression, for example under a particular condition. Probes are also used to detect products of amplification by polymerase chain reaction. The products of the reaction are hybridized to the probe and hybrids are detected. Probes are used for in situ hybridization to cells to detect expression. Probes can also be used in vivo for diagnostic detection of hybridizing sequences. Probes are typically labeled with a radioactive isotope. Other types of detectable labels may be used such as chromophores, fluors, and enzymes.

Expression of specific mRNA can vary in different cell types and can be tissue specific. This variation of mRNA levels in different cell types can be exploited with nucleic acid probe assays to determine tissue types. For example, PCR, branched DNA probe assays, or blotting techniques utilizing nucleic acid probes substantially identical or complementary to polynucleotides listed in the Sequence Listing can determine the presence or absence of cDNA or mRNA related to the polynucleotides of the invention.

Examples of a nucleotide hybridization assay are described in Urdea et al., PCT WO92/02526 and Urdea et al., U.S. Patent No. 5,124,246, both incorporated herein by reference. The references describe an example of a sandwich nucleotide hybridization assay.

Alternatively, the Polymerase Chain Reaction (PCR) is another means for detecting small amounts of target nucleic acids, as described in Mullis et al., Meth. Enzymol. (1987) 155:335-350; U.S. Patent No. 4,683,195; and U.S. Patent No. 4,683,202. Two primer polynucleotides nucleotides hybridize with the target nucleic acids and are used to prime the reaction. The primers may be composed of sequence within or 3' and 5' to the polynucleotides of the Sequence Listing. Alternatively, if the primers are 3' and 5' to these polynucleotides, they need not hybridize to them or the complements. A thermostable polymerase creates copies of target nucleic acids from the primers using the original target nucleic acids as a template. After a large amount of target nucleic acids is generated by the polymerase, it is detected by methods such as Southern blots. When using the Southern blot method, the labeled probe will hybridize to a polynucleotide of the Sequence Listing or complement.

Furthermore, mRNA or cDNA can be detected by traditional blotting techniques described in Sambrook et al., "Molecular Cloning: A Laboratory Manual" (New York, Cold Spring Harbor Laboratory, 1989). mRNA or cDNA generated from mRNA using a polymerase enzyme can be purified and separated using gel electrophoresis. The nucleic acids on the gel are then blotted onto a solid support, such as nitrocellulose. The solid support is exposed to a labeled probe and then washed to remove any unhybridized probe. Next, the duplexes containing the labeled probe are detected. Typically, the probe is labeled with radioactivity.

### Mapping

Polynucleotides of the present invention are used to identify a chromosome on which the corresponding gene resides. Using fluorescence in situ hybridization (FISH) on normal metaphase spreads, comparative genomic hybridization allows total genome assessment of changes in relative copy number of DNA sequences. See Schwartz and Samad, Current Opinions in Biotechnology (1994) 8:70-74; Kallioniemi et al., Seminars in Cancer Biology (1993) 4:41-46; Valdes and Tagle, Methods in Molecular Biology (1997) 68:1, Boultwood, ed., Human Press, Totowa, NJ.

Preparations of human metaphase chromosomes are prepared using standard cytogenetic techniques from human primary tissues or cell lines. Nucleotide probes comprising at least 12 contiguous nucleotides selected from the nucleotide sequence shown in the Sequence Listing are used to identify the corresponding chromosome. The nucleotide probes are labeled, for example, with a radioactive, fluorescent, biotinylated, or chemiluminescent label, and detected by well known methods appropriate for the particular label selected. Protocols for hybridizing nucleotide probes to preparations of metaphase chromosomes are also well known in the art. A nucleotide probe will hybridize specifically to nucleotide sequences in the chromosome preparations that are complementary to the nucleotide sequence of the probe. A probe that hybridizes specifically to a polynucleotide-related gene provides a detection signal at least 5-, 10-, or 20-fold higher than the background hybridization provided with non-EST coding sequences.

Polynucleotides are mapped to particular chromosomes using, for example, radiation hybrids or chromosome-specific hybrid panels. See Leach et al., Advances in Genetics, (1995) 33:63-99; Walter et al., Nature Genetics (1994) 7:22-28; Walter and Goodfellow, Trends in Genetics (1992) 9:352. Such mapping can be useful in identifying the function of the polynucleotide-related gene by its proximity to other genes with known function. Function can also be assigned to the related gene when particular syndromes or diseases map to the same chromosome.

### Tissue Profiling

The polynucleotides of the present invention can be used to determine the tissue type from which a given sample is derived. For example, a metastatic lesion is identified by its developmental organ or tissue source by identifying the expression of a particular marker of that organ or tissue. If a polynucleotide is expressed only in a specific tissue type, and a metastatic lesion is found to express that polynucleotide, then the developmental source of the lesion has been identified. Expression of a particular polynucleotide is assayed by detection of either the corresponding mRNA or the protein product. Immunological methods, such as antibody staining, are used to detect a particular protein product. Hybridization methods may be used to detect particular mRNA species, including but not limited to in situ hybridization and Northern blotting.

### Use of Polymorphisms

A polynucleotide will be useful in forensics, genetic analysis, mapping, and diagnostic applications if the corresponding region of a gene is polymorphic in the human population. A particular polymorphic form of the polynucleotide may be used to either identify a sample as deriving from a suspect or rule out the possibility that the sample derives from the suspect. Any means for detecting a polymorphism in a gene are used, including but not limited to electrophoresis of protein polymorphic variants, differential sensitivity to restriction enzyme cleavage, and hybridization to an allele-specific probe.

### Use of Polynucleotides to Raise Antibodies

Expression products of a polynucleotide, the corresponding mRNA or cDNA, or the corresponding complete gene are prepared and used for raising antibodies for experimental, diagnostic, and therapeutic purposes. The polynucleotide or related cDNA is expressed as described above, and antibodies are prepared. These antibodies are specific to an epitope on the polynucleotide-encoded polypeptide, and can precipitate or bind to the corresponding native protein in a cell or tissue preparation or in a cell-free extract of an in vitro expression system.

Immunogens for raising antibodies are prepared by mixing the polypeptides encoded by the polynucleotide of the present invention with adjuvants. Alternatively, polypeptides are made as fusion proteins to larger immunogenic proteins. Polypeptides are also covalently linked to other larger immunogenic proteins, such as keyhole limpet hemocyanin. Immunogens are typically administered intradermally, subcutaneously, or intramuscularly. Immunogens are administered to experimental animals such as rabbits, sheep, and mice, to generate antibodies. Optionally, the animal spleen cells are isolated and fused with myeloma cells to form hybridomas which secrete monoclonal antibodies. Such methods are well known in the art. According to another method known in the art, the polynucleotide is administered directly, such as by intramuscular injection, and expressed in vivo. The expressed protein generates a variety of protein-specific immune responses, including production of antibodies, comparable to administration of the protein.

Preparations of polyclonal and monoclonal antibodies specific for polynucleotide-encoded proteins and polypeptides are made using standard methods known in the art. The antibodies specifically bind to epitopes present in the polypeptides encoded by polynucleotides disclosed in the Sequence Listing. Typically, at least 6, 8, 10, or 12 contiguous amino acids are required to form an epitope. However, epitopes which involve non-contiguous amino acids may require more, for example at least 15, 25, or 50 amino acids. A short sequence of a polynucleotide may then be unsuitable for use as an epitope to raise antibodies for identifying the corresponding novel protein, because of the potential for cross-reactivity with a known protein. However, the antibodies may be useful for other purposes, particularly if they identify common structural features of a known protein and a novel polypeptide encoded by a polynucleotide of the invention.

Antibodies that specifically bind to human polynucleotide-encoded polypeptides should provide a detection signal at least 5-, 10-, or 20-fold higher than a detection signal provided with other proteins when used in Western blots or other immunochemical assays. Preferably, antibodies that specifically bind polypeptides do not detect other proteins in immunochemical assays and can immunoprecipitate EST-encoded proteins from solution. For such immunoassays, any type of samples can be used, including tissue, organs, cells, urine, blood, prostatic fluid or semen.

Of interest are antibodies to the secreted polypeptides encoded by the polynucleotide sequences of the present invention. Antibodies to secreted polypeptides can be used to test body fluids, such as blood, urine, prostatic fluid and semen.

To test for the presence of serum antibodies to the polypeptide in a human population, human antibodies are purified by methods well known in the art. Preferably, the antibodies are affinity purified by passing antiserum over a column to which a protein, polypeptide, or fusion protein is bound. The bound antibodies can then be eluted from the column, for example using a buffer with a high salt concentration.

In addition to the antibodies discussed above, genetically engineered antibody derivatives are made, such as single chain antibodies or humanized antibodies.

Antibodies were prepared using two sequences from clone SL-5: H₂N-CGPRLPSFPCPTHEPSTGQLSK-CONH₂ and H₂N-CKDSQGLSDFKR-NSRTTRRSYKCCONH₂. Using polyclonal antibodies raised against a mixture of these polypeptides, immunohistochemistry was performed on a variety of tumor tissues and corresponding normal tissue. The results are shown in Figure 3, and discussed in the Examples. These polypeptides are useful for detecting a higher level of expression of clone SL-5 in tumor tissues.

### Use of Polynucleotides to Construct Arrays for Diagnostics

The present polynucleotide sequences and gene products are useful for determining the occurrence of cancer, tumor progression, hyperproliferative growth, and/or accompanying biological or physical manifestations. Specifically, the polynucleotides of the instant invention can be utilized to determine the occurrence of prostatic disorders, such as BPH or localized prostate cancer.

A number of prostatic disorders exist, including adenocarcinoma, BPH, histologic prostate cancer, prostatic intraepithelial neoplasia, clinical prostate cancer, incidental prostate cancer, and localized prostate cancer. BPH is a common prostatic disorder in men which becomes clinically manifest usually after age fifty. In BPH, hyperplastic growth of prostatic cells in the periurethral glandular tissue in the central zone of the prostate gland cause an enlarged prostate which can compress or elongate the urethra and produce symptoms of urethral obstruction that may progress to urinary retention or to a constellation of symptoms known as prostatism. A host of physical manifestations can accompany prostatic disorders including: impotency, reduced urinary flow, hesitancy in initiating voiding, postvoid dribbling, a sensation of incomplete bladder emptying, and development of bladder or high urinary tract infections.

To determine the occurrence of prostate cancer, prostate tumour progression, hyperproliferative growth prostate cell and/or accompanying biological or physical manifestations, the levels of polynucleotides of the present invention in a sample are compared to the levels in a normal control of body tissues, cells, organs, or fluids. The normal control can include a pool of cells from a particular organ or tissue or tissues and/or cells from throughout the body. Either the immunoassays described above or the nucleic acid assays described below can be used for such measurements.

Any observed difference between the sample and normal control can indicate the occurrence of disease or disorder. Typically, if the levels of the polynucleotides of the present invention are higher than those found in the normal control, the results indicate the occurrence of prostate cancer, prostate tumor progression, hyperproliferative prostate cell growth, and/or accompanying biological or physical manifestations. In addition, the present polynucleotides can be useful to diagnose the

In addition, the present polynucleotides can be useful to diagnose the severity as well as the occurrence of prostate cancer, prostate tumor progression, hyperproliferative prostate cell growth, and/or accompanying biological or physical manifestations, including prostatic disorders. For example, the greater the difference observed in the sample versus the normal control of the present polynucleotides or encoded polypeptides, the greater the severity of the disorder, in particular, when higher levels as compared to a normal control are observed.

The polynucleotides of the present invention, were expressed at higher levels in a prostate cancer cell line versus a normal prostate epithelial cell line.

Polynucleotide arrays provide a high throughput technique that can assay a large number of polynucleotide sequences in a sample. This technology can be used as a diagnostic and as a tool to test for differential expression to determine function of an encoded protein.

To create arrays, polynucleotide probes are spotted onto a substrate in a two-dimensional matrix or array. Samples of polynucleotides can be labeled and then hybridized to the probes. Double stranded polynucleotides, comprising the labeled sample polynucleotides bound to probe polynucleotides, can be detected once the unbound portion of the sample is washed away.

The probe polynucleotides can be spotted on substrates including glass, nitrocellulose, etc. The probes can be bound to the substrate by either covalent bonds or by non-specifc interactions, such as hydrophobic interactions. The sample polynucleotides can be labeled using radioactive labels, fluorophors, etc.

Techniques for constructing arrays and methods of using these arrays are described in EP No. 0 799 897; PCT No. WO 97/29212; PCT No. WO 97/27317; EP No. 0 785 280; PCT No. WO 97/02357; U.S. Pat. No. 5,593,839; U.S. Pat. No. 5,578,832; EP No. 0 728 520; U.S. Pat. No. 5,599,695; EP No. 0 721 016; U.S. Pat. No. 5,556,752; PCT No. WO 95/22058; and U.S. Pat. No. 5,631,734.

Further, arrays can be used to examine differential expression of genes and can be used to determine gene function. For example, arrays of the instant polynucleotide sequences can be used to determine if any of the EST sequences are differentially expressed between normal cells and cancer cells, for example. High expression of a particular message in a cancer cell, which is not observed in a corresponding normal cell, can indicate a cancer specific protein.

### Differential Expression

Also described is a method to identify abnormal or diseased tissue in a human. For polynucleotides corresponding to profiles of protein families as described above, the choice of tissue may be dictated by the putative biological function. The expression of a gene corresponding to a specific polynucleotide is compared between a first tissue that is suspected of being diseased and a second, normal tissue of the human. The normal tissue is any tissue of the human, especially those that express the polynucleotide-related gene including, but not limited to, brain, thymus, testis, heart, prostate, placenta, spleen, small intestine, skeletal muscle, pancreas, and the mucosal lining of the colon.

The polynucleotide-related genes in the two tissues are compared by any means known in the art. For example, the two genes are sequenced, and the sequence of the gene in the tissue suspected of being diseased is compared with the gene sequence in the normal tissue. The polynucleotide-related genes, or portions thereof, in the two tissues are amplified, for example using nucleotide primers based on the nucleotide sequence shown in the Sequence Listing, using the polymerase chain reaction. The amplified genes or portions of genes are hybridized to nucleotide probes selected from the same nucleotide sequence shown in the Sequence Listing. A difference in the nucleotide sequence of the polynucleotide-related gene in the tissue suspected of being diseased compared with the normal nucleotide sequence suggests a role of the polynucleotide-encoded proteins in the disease, and provides a lead for preparing a therapeutic agent. The nucleotide probes are labeled by a variety of methods, such as radiolabeling, biotinylation, or labeling with fluorescent or chemiluminescent tags, and detected by standard methods known in the art.

Alternatively, polynucleotide-related mRNA in the two tissues is compared. PolyA⁺ RNA is isolated from the two tissues as is known in the art. For example, one of skill in the art can readily determine differences in the size or amount of polynucleotide-related mRNA transcripts between the two tissues using Northern blots and nucleotide probes selected from the nucleotide sequence shown in the Sequence Listing. Increased or decreased expression of an polynucleotide-related mRNA in a tissue sample suspected of being diseased, compared with the expression of the same polynucleotide-related mRNA in a normal tissue, suggests that the expressed protein has a role in the disease, and also provides a lead for preparing a therapeutic agent.

Any method for analyzing proteins is used to compare two polynucleotide-encoded proteins from matched samples. The sizes of the proteins in the two tissues are compared, for example, using antibodies of the present invention to detect polynucleotide-encoded proteins in Western blots of protein extracts from the two tissues. Other changes, such as expression levels and subcellular localization, can also be detected immunologically, using antibodies to the corresponding protein. A higher or lower level of polynucleotide-encoded protein expression in a tissue suspected of being diseased, compared with the same polynucleotide-encoded protein expression level in a normal tissue, is indicative that the expressed protein has a role in the disease, and provides another lead for preparing a therapeutic agent.

Similarly, comparison of polynucleotide gene sequences or of polynucleotide gene expression products, e.g., mRNA and protein, between a human tissue that is suspected of being diseased and a normal tissue of a human, are used to follow disease progression or remission in the human. Such comparisons of polynucleotide-related genes, mRNA, or protein are made as described above.

For example, increased or decreased expression of the polynucleotide-related gene in the tissue suspected of being neoplastic can indicate the presence of neoplastic cells in the tissue. The degree of increased expression of the polynucleotide gene in the neoplastic tissue relative to expression of the gene in normal tissue, or differences in the amount of increased expression of the polynucleotide gene in the neoplastic tissue over time, is used to assess the progression of the neoplasia in that tissue or to monitor the response of the neoplastic tissue to a therapeutic protocol over time. The expression pattern of any two cell types can be compared, such as low and high metastatic tumor cell lines, or cells from tissue which have and have not been exposed to a therapeutic agent.

### Computer-Related Embodiments

In general, a library of polynucleotides is a collection of sequence information, which information is provided in either biochemical form (e.g., as a collection of polynucleotide molecules), or in electronic form (e.g., as a collection of polynucleotide sequences stored in a computer-readable form, as in a computer system and/or as part of a computer program). The sequence information of the polynucleotides can be used in a variety of ways, *e.g*., as a resource for gene discovery, as a representation of sequences expressed in a selected cell type (e.g., cell type markers), and/or as markers of a given disease or disease state. In general, a disease marker is a representation of a gene product that is present in all cells affected by disease either at an increased or decreased level relative to a normal cell (e.g., a cell of the same or similar type that is not substantially affected by disease).

The nucleotide sequence information of the library can be embodied in any suitable form, e.g., electronic or biochemical forms. For example, a library of sequence information embodied in electronic form comprises an accessible computer data file (or, in biochemical form, a collection of nucleic acid molecules) that contains the representative nucleotide sequences of genes that are differentially expressed (e.g., overexpressed or underexpressed) as between, for example, a cancerous cell and a normal cell. Biochemical embodiments of the library include a collection of nucleic acids that have the sequences of the genes in the library, where the nucleic acids can correspond to the entire gene in the library or to a fragment thereof, as described in greater detail below.

The polynucleotide libraries described herein generally comprise sequence information of a plurality of polynucleotide sequences, where at least one of the polynucleotides is a polynucleotide of the present invention. By plurality is meant at least 2, usually at least 3 and can include up to all of the polynucleotides of the present invention. The length and number of polynucleotides in the library wilt vary with the nature of the library, e.g., if the library is an oligonucleotide array, a cDNA array, a computer database of the sequence information, etc.

Where the library is an electronic library, the nucleic acid sequence information can be present in a variety of media. "Media" refers to a manufacture, other than an isolated nucleic acid molecule, that contains the sequence information of the present invention. Such a manufacture provides the genome sequence or a subset thereof in a form that can be examined by means not directly applicable to the sequence as it exists in a nucleic acid. For example, the nucleotide sequence e.g., the nucleic acid sequences of any of the polynucleotides of of the present invention, can be recorded on computer readable media, *e.g*., any medium that can be read and accessed directly by a computer. Such media include, but are not limited to: magnetic storage media, such as a floppy disc, a hard disc storage medium, and a magnetic tape; optical storage media such as CD-ROM; electrical storage media such as RAM and ROM; and hybrids of these categories such as magnetic/optical storage media. One of skill in the art can readily appreciate how any of the presently known computer readable mediums can be used to create a manufacture comprising a recording of the present sequence information. "Recorded" refers to a process for storing information on computer readable medium, using any such methods as known in the art. Any convenient data storage structure can be chosen, based on the means used to access the stored information. A variety of data processor programs and formats can be used for storage, *e.g*., word processing text file, database format, *etc.* In addition to the sequence information, electronic versions of the libraries can be provided in conjunction or connection with other computer-readable information and/or other types of computer-readable files (*e.g.,* searchable files, executable files, *etc,* including, but not limited to, for example, search program software, *etc.*).

By providing the nucleotide sequence in computer readable form, the information can be accessed for a variety of purposes. Computer software to access sequence information is publicly available. For example, the BLAST (Altschul et al., *supra.*) and BLAZE (Brutlag et al. *Comp. Chem.* (1993) 17:203) search algorithms on a Sybase system can be used to identify open reading frames (ORFs) within the genome that contain homology to ORFs from other organisms.

As used herein, "a computer-based system" refers to the hardware means, software means, and data storage means used to analyze the nucleotide sequence information of the present invention. The minimum hardware of the computer-based systems described herein comprises a central processing unit (CPU), input means, output means, and data storage means. A skilled artisan can readily appreciate that any one of the currently available computer-based system are suitable for use in the present invention. The data storage means can comprise any manufacture comprising a recording of the present sequence information as described above, or a memory access means that can access such a manufacture.

"Search means" refers to one or more programs implemented on the computer-based system, to compare a target sequence or target structural motif, or expression levels of a polynucleotide in a sample, with the stored sequence information. Search means can be used to identify fragments or regions of the genome that match a particular target sequence or target motif. A variety of known algorithms are publicly known and commercially available, *e.g*., MacPattern (EMBL), BLASTN and BLASTX (NCBI). A "target sequence" can be any polynucleotide or amino acid sequence of six or more contiguous nucleotides or two or more amino acids, preferably from about 10 to 100 amino acids or from about 30 to 300 nt A variety of comparing means can be used to accomplish-comparison of sequence information from a sample (*e.g.,* to analyze target sequences, target motifs, or relative expression levels) with the data storage means. A skilled artisan can readily recognize that any one of the publicly available homology search programs can be used as the search means for the computer based systems described herein to accomplish comparison of target sequences and motifs. Computer programs to analyze expression levels in a sample and in controls are also known in the art.

A "target structural motif," or "target motif," refers to any rationally selected sequence or combination of sequences in which the sequence(s) are chosen based on a three-dimensional configuration that is formed upon the folding of the target motif, or on consensus sequences of regulatory or active sites. There are a variety of target motifs known in the art. Protein target motifs include, but arc not limited to, enzyme active sites and signal sequences. Nucleic acid target motifs include, but are not limited to, hairpin structures, promoter sequences and other expression elements such as binding sites for transcription factors.

A variety of structural formats for the input and output means can be used to input and output the information in the computer-based systems of the present invention. One format for an output means ranks the relative expression levels of different polynucleotides. Such presentation provides a skilled artisan with a ranking of relative expression levels to determine a gene expression profile..

As discussed above, the "library" described herein also encompasses biochemical libraries of the polynucleotides of the invention *e.g.*, collections of nucleic acids representing the provided polynucleotides. The biochemical libraries can take a variety of forms, *e.g.*, a solution of cDNAs, a pattern of probe nucleic acids stably associated with a surface of a solid support (*i.e.*, an array) and the like. Of particular interest are nucleic acid arrays in which one or more of the polynucleotides of the invention is represented on the array. By array is meant a an article of manufacture that has at least a substrate with at least two distinct nucleic acid targets on one of its surfaces, where the number of distinct nucleic acids can be considerably higher, typically being at least 10 nt, usually at least 20 nt and often at least 25 nt. A variety of different array formats have been developed and are known to those of skill in the art. The arrays of the subject invention find use in a variety of applications, including gene expression analysis, drug screening, mutation analysis and the like, as disclosed in the above-listed exemplary patent documents.

In addition to the above nucleic acid libraries, analogous libraries of polypeptides are also provided, where the where the polypeptides of the library will represent at least a portion of the polypeptides encoded by the polynucleotides of the invention.

The present invention will now be illustrated by reference to the following examples which set forth particularly advantageous embodiments. However, it should be noted that these embodiments are illustrative and are not to be construed as restricting the invention in any way.

### EXAMPLES

### EXAMPLE 1

### ISOLATION OF THE POLYNUCLEOTIDES

cDNA libraries were prepared from PrEC, normal human prostate epithelial cells, and LNCaP, a cell line derived from human lymph node metastasized prostate cancer. PrEC cells are available from Clonetics, San Diego, California, U.S.A. LNCaP cells are available from the ATCC, Manassas, Virginia, U.S.A.

Using a PCR technique and reagents available from Clontech, Palo Alto, California, USA (CLONTECH PCR-Select™), mRNA up-regulated in LNCaP was captured and amplified. The captured polynucleotide inserts were inserted in the pCR2.1 vector, available from Invitrogen, Carlsbad, California, U.S.A. The vectors with the inserts were transformed into *E*. *coli* cells.

### EXAMPLE 2

### CONFIRMATION OF DIFFERENTIAL DISPLAY

Ten clones were chosen at random, and up-regulation of the sequences of these clone inserts in LNCaP versus PrEC cells was confirmed by Northern blot. Dot blots were performed on 168 clones and up-regulation was confirmed.

Further, sequencing of the clones showed that prostate specific antigen (PSA) and prostate specific membrane antigen (PSMA) sequences were isolated by the process described in Example 1. A good correlation between increased serum PSA levels and prostate tumors has been observed. PSMA, a cell surface antigen, is another observed marker for prostate cancer. See Bosland, Encyclopedia of Cancer, Volume II, pages 1283-1296 (1997), Academic Press. Thus, the data confirm that up-regulated mRNA characteristic of gene expression in prostate cancer was cloned by the method of Example 1.

### EXAMPLE 3

### POLYNUCLEOTIDE SEQUENCES

Some of the sequence results are shown in SEQ ID NO:218, 219 or 335. For the sequencing experiments, each clone was named SL-1 to SL-209. Each sequence was designated a unique combination of two names. This unique combination is shown in Table 1 in columns 2 and 3, denoted as "Sequence Name" and "Other Seq Name."

Table 1 indicates all the sequences that correspond to clone SL - 68.

Clones also were assigned cluster numbers. See column 4 of Table 1. Clones with the same cluster number generally comprise sequence derived from the same mRNA transcripts.

The last column of Table I indicates the nearest neighbor as determined by an alignment to sequences in a publicly available database.

A consensus for the sequence of each clone can be constructed by aligning the corresponding sequences or reverse complements thereof. Table 1 lists the names of all the sequences that correspond to each clone, and Table 2 shows the specific sequence that corresponds to each unique combination of Sequence Name and/or "Other Seq. Name."

The entire insert of some clones may not be represented by the sequences presented in Table 2. For example, the 5' and 3' ends of a clone insert may have been sequenced, but the sequences do not overlap. Additional sequence corresponding to the clone insert can be isolated and determined by constructing probes or primers from the sequences presented in Table 2 and a library of mRNA or cDNA from a prostate cell or prostate cancer cell line using the methods described above.

### EXAMPLE 4

### RESULTS OF PUBLIC DATABASE SEARCH

Both the nucleotide sequence and translations of masked sequences shown in the Sequence Listing were aligned with individual sequences that were publicly available. Similarity with individual sequences is used to determine the activity of the polypeptides encoded by genes corresponding to the sequences referred to in Table 2.

The sequences in SEQ ID NO: 218 or 219 first were masked to remove the pCR2.1 vector sequences. Masking was performed by aligning the pCR2.1 sequences with each of SEQ ID NO: 218 or 219 using the BLASTN program. Any sequence that produced an alignment with a score of less that 0.1 was masked.

A BLASTN vs. Genbank search was performed using the masked sequences with search parameters of greater than 99% overlap, 99% identity, and a p value of less than 1 x 10⁻⁴⁰ and this resulted in discard of sequences. Sequences from this search also were discarded if the inclusive parameters were met, but the sequence was ribosomal or vector-derived.

The resulting sequences from the previous search were classified into three groups (1, 2 and 3 below) and searched in a BLASTX vs. NRP (non-redundant proteins) database search: (I) unknown (no hits in the Genbank search), (2) weak similarity (greater than 45% identity and p value of less than 1 x 10⁻⁵), and (3) high similarity (greater than 60% overlap, greater than 80% identity, and p value less than 1 x 10⁻⁵). This search resulted in discard of sequences as having greater than 99% overlap, greater than 99% identity, and p value of less than I x 10⁻⁴⁰.

The remaining sequences were classified as unknown (no hits), weak similarity, and high similarity (parameters as above). Two searches were performed on this set of sequences. First, a BLAST vs. EST database search resulted in discard of sequences with greater than 99% overlap, greater than 99% similarity and a p value of less than 1 x 10⁻⁴⁰; sequences with a p value of less than 1 x 10⁻⁶⁵ when compared to a database sequence of human origin were also excluded. Second, a BLASTN vs. Patent GeneSeq database resulted in discard of sequences with greater than 99% identity; p value less than 1 x 10⁻⁴⁰; greater than 99% overlap.

The masked sequences were translated in all six reading frames to determine the best alignment with the individual sequences. These amino acid sequences and nucleotide sequences are referred, generally, as query sequences, which are aligned with the individual sequences.

Query and individual sequences were aligned using the BLAST programs, available over the world wide web.

Table 2 shows the results of the alignments. Table 2 refers to each sequence by its Sequence Name and/or "Other Seq. Name" and includes the accession numbers and descriptions of nearest neighbors from the Genbank and Non-Redundant Protein searches.

The activity of the polypeptide encoded by the sequences referred to in Table 2 is expected to be the same or similar to the nearest neighbor reported in Table 2. The accession number of the nearest neighbor is reported, providing a reference to the activities exhibited by the nearest neighbor. The search program and database used for the alignment also are indicated as well as a calculation of the p value.

Full length sequences or fragments of the polynucleotide sequences of the nearest neighbors can be used as probes and primers to identify and isolate the full length sequence corresponding to sequence referred to in Table 2. Although full length sequences can be obtained from the cell lines described above, the nearest neighbors can indicate a tissue or cell type to be used to construct a library for the full-length sequences of those referred to in Table 2.

The sequences referred to in Table 2 and the translations thereof may be human homologs of known genes of other species or novel allelic variants of known human genes. In such cases, these new human sequences may be suitable as diagnostics, prognostics, or therapeutics. As diagnostics, the human sequences exhibit greater specificity in detecting and differentiating human cell lines and types than homologs of other species. The human polypeptides are less likely to be immunogenic when administered to humans than homologs from other species. Further, on administration to humans, the encoded polypeptides can show greater specificity or can be better regulated by other human proteins than are homologs from other species.

In the preferred embodiments of the invention, the sequences shown in SEQ ID NO: 218, 219 or 335 consisting of the unmasked regions should be considered as the source of probes and primers, as these sequences are most representative of the distinguishing portions of these polynucleotides.

Generally, the masking itself does not influence the search results as shown in Table 2, except to eliminate multiple "hits" based on similarity to repetitive regions common to more than one polypeptide.

### EXAMPLE 5

### ANALYSIS OF CLONE

### Clone SL-68 (SEQ ID NO:218 and 219)

Two transcripts, 2.6kb and 4.3kb, were observed in normal spleen, thymus and peripheral blood leukocytes, as well as in promyelocytic leukemia, chronic myelogenous leukemia and lymphoblastic leukemia. The 4.3kb transcript was seen in normal testis, colon, Hela cell S3, colorectal adenocarcinoma and melanoma. The 2.6kb band was found in the following prostate cell lines: PC-3 (metastatic to bone, androgen insensitive); DU-145 (metastatic to brain, androgen insensitive); FFpz (primary cells derived from normal prostate epithelium); Ffca (primary cells derived from Gleason Grade 3 prostate cancer epithelium); and WO-CA (primary cells derived from Gleason Grade 4 prostate cancer epithelium). However, higher expression was observed in LNCaP, MDA PCa 2A, HPV-7 and HPV-10. A 9.5kb transcript was also observed in MDA PCa 2A and 2B. Additional sequence corresponding to this clone is disclosed in SEQ ID NO:335.

Those skilled in the art will recognize, or be able to ascertain, using not more than routine experimentation, many equivalents to the specific embodiments of the invention described herein. Such specific embodiments and equivalents are intended to be encompassed by the following claims.

**TABLE 1**

| Clone # | Sequence Name | Other Seq Name | Clone # | Nearest Neighbor If Available |
|---|---|---|---|---|
| | | | Cluster # | |
| SL-068 | SL068m13 | WE97.B4.M13 | SL-068 | |
| | SL068t7 | WE97.B4.T7 | | |

**TABLE 2**

| | BlastN vs. Gb (nearest neighbour) | | | BlastX vs. NRPdb (nearest neighbour) | | |
|---|---|---|---|---|---|---|
| Seq. Name and/or Other Seq. Name | Accession | Hit Description | P(V) | Accession | Hit Description | P(V) |
| SL68m13 | AC004157 | ***SEQUENCING IN PROGRESS | 0.00071 | <NONE> | <NONE> | <NONE> |
| | | ***Plasmodium falciparum 3D7 chromosome 12 PFYAC293 genomic sequence; HTGS phase 1, 18 unordered pieces. | | | | |
| SL68t7 | AJ226619 | Ciona intestinalis genomic fragment, clone 17H6, genomic survey sequence. | 0.064 | <NONE> | <NONE> | <NONE> |

## Claims

1. A method of diagnosing prostate cancer, prostate tumour progression and hyperproliferative prostate cell growth comprising:
(a) providing a polynucleotide probe comprising at least 20 contiguous nucleotides of SEQ ID NO: 218, 219 or 335 or a complement thereof;
(b) contacting a biological sample for diagnosis with said probe under hybridizing conditions that permit formation of a duplex; and
(c) determining the presence of said duplex, and detecting the differences that exist between the levels of duplexes in said biological sample as compared to a normal biological sample.

2. A diagnostic kit comprising:
(a) a diagnostic regeant comprising a polynucleotide probe comprising at least 20 contiguous nucleotides of SEQ ID NO: 218, 219 or 335 or a complement thereofwhen said sequence is present in a test biological sample;
(b) a normal biological sample; and
(c) instructions for detecting the differences that exist between the levels of duplexes in said test biological sample as compared to said normal biological sample.

3. An isolated polynucleotide selected from the group consisting of: a polynucleotide comprising the nucleotide sequence of SEQ ID NO: 218 or 219.

4. A vector comprising the polynucleotide of claim 3.

5. An isolated host cell comprising the vector of claim 4.

## Patentansprüche

1. Verfahren zum Diagnostizieren von Prostatakrebs, Prostatatumorprogression und hyperproliferativem Prostatazellwachstum, umfassend:
(a) Bereitstellung einer Polynukleotidsonde, umfassend mindestens 20 zusammenhängende Nukleotide von SEQ ID NO: 218, 219 oder 335 oder ein Komplement davon;
(b) Inkontaktbringen einer biologischen Probe zur Diagnose mit besagter Sonde unter hybridisierenden Bedingungen, welche die Bildung einer Duplex erlauben und
(c) Bestimmung der Gegenwart besagter Duplex und Detektion der Unterschiede, welche zwischen den Leveln von Duplices in besagter biologischer Probe existieren im Vergleich zu einer normalen biologischen Probe.

2. Diagnosekit, welches umfasst:
(a) ein diagnostisches Reagens, umfassend eine Polynukleotidsonde, umfassend mindestens 20 zusammenhängende Nukleotide von SEQ ID NO: 218, 219 oder 335 oder ein Komplement davon, wenn besagte Sequenz in einer biologischen Testprobe vorhanden ist;
(b) eine normale biologische Probe und
(c) Anleitungen für die Detektion der Unterschiede, welche zwischen den Leveln von Duplices in besagter biologischer Testprobe existieren im Vergleich zu besagter normaler biologischen Probe.

3. Isoliertes Polynukleotid, ausgewählt aus der Gruppe, bestehend aus einem Polynukleotid, umfassend die Nukleotidsequenz von SEQ ID NO: 218 oder 219.

4. Vektor, umfassend das Polynukleotid nach Anspruch 3.

5. Isolierte Wirtszelle, welche den Vektor nach Anspruch 4 umfasst.

## Revendications

1. Procédé de diagnostic d'un cancer de la prostate, de l'évolution d'une tumeur de la prostate et d'une croissance de cellules hyperprolifératives de la prostate, comprenant les étapes consistant à :
(a) prendre une sonde polynucléotidique comprenant au moins 20 nucléotides contigus de la SEQ ID NO : 218, 219 ou 335 ou un complément de celle-ci ;
(b) mettre un échantillon biologique, destiné au diagnostic, en contact avec ladite sonde dans des conditions d'hybridation qui permettent la formation d'un duplex ; et
(c) déterminer la présence dudit duplex, et détecter les différences qui existent entre les niveaux de duplex dans ledit échantillon biologique par rapport à un échantillon biologique normal.

2. Kit de diagnostic comprenant :
(a) un réactif de diagnostic comprenant une sonde polynucléotidique comprenant au moins 20 nucléotides contigus de la SEQ ID NO : 218, 219 ou 335 ou un complément de celle-ci lorsque ladite séquence est présente dans un échantillon biologique à tester ;
(b) un échantillon biologique normal ; et
(c) des instructions pour détecter les différences qui existent entre les niveaux de duplex dans ledit échantillon biologique à tester par rapport audit échantillon biologique normal.

3. Polynucléotide isolé choisi parmi le groupe comprenant : un polynucléotide comprenant la séquence nucléotidique SEQ ID NO : 218 ou 219.

4. Vecteur comprenant le polynucléotide selon la revendication 3.

5. Cellule hôte isolée comprenant le vecteur selon la revendication 4.


### SEQUENCE LISTING

<110> Zhang, Jimmy
   Aste1, Jon H.
   Carroll III, Eddie
   Endege, wilson O.
   Pord, Donna M.
   Monahan, John B.
   Schlegel, Robert
   Steinmann, Kathleen E.
<120> GENES AND GENE EXPRESSION PRODUCTS THAT ARE DIFFERENTIALLY REGULATED IN PROSTATE CANCER
<130> 200130.463
<140> US
   <141> 1999-06-11
<160> 3
<170> FastSEQ for Windows Version 3.0
<210> 218
   <211> 498
   <212> DNA
   <213> Homo Sapien
<400> 218
   cacttgacct gattacgcca agcttggtac cgagctcgga tccactagta acggccgcca      60
   gtgtgctgga attcgccctt tcgagcggcc gcccgggcag gtactttttt tttttttttt     120
   taattcccac aacaacccat ttcaaaatga gaaaactagg ttgagtgact tgtccacagt     180
   tccaaagcta ataaaaatga tgaggcatat ttctcttctg ggcccactgt attcagttct     240
   ttgttcttta cactgagtgc cgaaaaaaaa aaatcagact attttgattc tagaaagtga     300
   ttttggtata ataaaggagt ttgaagaaac aaatgacatc attcctgatg atggtagccc     360
   actccaacaa aggcgtatat atgtaggcaa gtttgaagat atctataaga gcattaaaag     420
   actccaacaa aggcgtatat atgtaggcaa gtttgaagat atctataaga gcattaaaag     480
   gcaagtgcac cattgtgg                                                   498
<210> 219
   <211> 818
   <212> DNA
   <213> Homo Sapien
<220>
   <221> misc_feature
   <222> (1)...(818)
   <223> n = A,T,C or G
<400> 219
   ggcctntnga gctgctcgac ggccgccatg tgatggatat ctgcagaatt cgcccttagc      60
   gtggcgcggc cgaggtacct agaaaacaga aacttgagta gacatggtaa tgaccagaaa     120
   aggctatctt tatacatttc ttttgctacg cttcaaattc atgtcaccta aaagttgtga     180
   agtgcacaaa acaaatctac ttaactgaaa attattttca atgaatggga tgtttagaac     240
   tctgtgaggg tttttaaggt cttttcgaat agcaaattct aatgaggctt ttttaagttg     300
   gcaatttaaa ctcatacaag aaataaaaac tcaccagtgt ggctgggcag aatatatata     360
   ttttctcaaa tattgtttgt ttgttttttc cctgcactgt atccatggtc ccatgatgaa      420
   actgttatat tgctgatata tttattggaa tatgtgggcc aacttccttt ccactcaaca     480
   tatggattgg tagtttaaaa taattccttt ctattaagca aatgtgtggc taaggcacat     540
   ttaaatagcc cattaaacca atgagatgac aatgtgttac cctcagagaa agcttaattt     600
   ttggagtaat caattacaca tatcacagaa tgtctcatga gaacattttt ggctaggtct     660
   accaatttat catgcaaata attatagatt ttcatttgag gcaaagatgc tgattcatca     720
   ttagtaacat ggtcacaaat aatcatttat tttattttgg taacatctgt ctttcctgtg     780
   gggaaactta ctatatgctc tacgttaatt aaattaaa                             818
<210> 335
   <211> 1312
   <212> DNA
   <213> Homo sapien
<400> 335
   acctagaaaa cagaaacttg agtagacatg gtaatgacca gaaaaggcta tctttataca      60
   tttcttttgc tacgcttcaa attcatgtca cctaaaagtt gtgaagtgca caaaacaaat     120
   ctacttaact gaaaattatt ttcaatgaat gggatgttta gaactctgtg agggttttta     180
   aggtcttttc gaatagcaaa ttctaatgag gcttttttaa gttggcaatt taaactcata    240
   caagaaataa aaactcacca gtgtggctgg gcagaatata tatattttct caaatattgt     300
   ttgtttgttt tttccctgca ctgtatccat ggtcccatga tgaaactgtt atattgctga     360
   tatatttatt ggaatatgtg ggccaacttc ctttccactc aacatatgga ttggtagttt     420
   aaaataattc ctttctatta agcaaatgtg tggctaaggc acatttaaat agcccattaa     480
   accaatgaga tgacaatgtg ttaccctcag agaaagctta atttttggag taatcaatta     540
   cacatatcac agaatgtctc atgagaacat ttttggctag gtctaccaat ttatcatgca     600
   aataattata gattttcatt tgaggcaaag atgctgattc atcattagta acatggtcac     660
   aaataatcat ttattttatt tttgttaaca tctgtctttc ctgtggggaa acttactata     720
   tgctctacgt ttatttaatt taaaaagtca attggttatt ctgaattttt aaaaataaca     780
   taaaactgtt gttctaaatc acagcacctg cttttctttt tttagtgaaa ttatataagc     840
   atttagagaa tgaaagtgta agacttgtgg tttctggtct ctttttactg tttgtaagcc     900
   tactcgtcat gatattccac aatggtgcac ttgcctttta atgctcttat agatatcttc     960
   aaacttgcct acatatatac gcctttgttg gagtgggcta ccatcatcag gaatgatgtc    1020
   atttgtttct tcaaactcct ttattatacc aaaaaagtga cagactccac agtctgatca    1080
   gttttggaga aatatgttaa cattttcaat tatctcactt tctagaatca aaatagtctg    1140
   attttttttt ttcggcactc agtgtaaaga acaaagaact gaatacagtg ggcccagaag    1200
   agaaatatgc ctcatcattt ttattagctt tggaactgtg gacaagtcac tcaacctagt    1260
   tttctcattt tgaaatgggt tgttgtggga attaaaaaaa aaaaaaaaaa gt            1312